# EUROPÄISCHE PATENTSCHRIFT

(11) **EP 0 456 153 B1**
(45) Veröffentlichungstag und Bekanntmachung des Hinweises auf die Patenterteilung: **05.04.1995**
(21) Anmeldenummer: 91107308.8
(22) Anmeldetag: 06.05.1991
(51) Int. Cl.: A61K 38/00, A61K 38/08, A61K 38/22, A61K 9/00, A61K 9/08, A61K 47/02

(54) **Galenische wässrige Formulierungen von Erythropoietin und ihre Verwendung**
Galenical aqueous formulations containing erythropoietin and use thereof
Formulations galéniques aqueuses à base d'érythropoiétine et leur utilisation

(30) Priorität: 08.05.1990 DE 4014654
(43) Veröffentlichungstag der Anmeldung: 13.11.1991
(73) Patentinhaber: BEHRINGWERKE Aktiengesellschaft, 35001 Marburg (DE)
(72) Erfinder: Brazel, Dieter, Dr., W-3550 Marburg (DE); Siebold, Bernhard, Dr., W-3550 Marburg (DE); Krumwieh, Dorothee, Dr., W-3550 Marburg (DE); Brune, Thomas, Dr., W-3550 Marburg (DE)

(56) Entgegenhaltungen:
- EP-A- 0 178 576
- EP-A- 0 178 665
- EP-A- 0 306 824

## Beschreibung

Die Erfindung betrifft galenische wäßrige Formulierungen von gereinigten Erythropoietin, insbesondere von humanem nativem und von rekombinantem humanem Erythropoietin (rh EPO). Die Formulierungen der Erfindung - ohne Fremdproteine, Zucker, Aminosäuren oder andere übliche Stabilisierungsmittel - behalten bei einer Temperatur von 4-8°C für mindestens 1 Jahr mindestens etwa 78 % ihrer ursprünglichen Aktivität.

Erythropoietin (EPO) ist ein Glykoprotein mit 166 Aminosäuren, 3 Glykosylierungsstellen an den Aminosäure-Positionen 24, 38 und 83 und einem Molekulargewicht von etwa 34 000. EPO läßt sich entweder aus natürlichen Quellen, wie menschlichem Urin isolieren (vgl. z.B. Miyake et al., J. Biol. Chem., Bd. 252 (1977), 5558-5564) oder es kann durch gentechnologische Verfahren hergestellt werden (vgl. z.B. EP-A 0148 605 und 0 267 678). Wäßrige Lösungen von Erythropoietin sind bei Temperaturen von etwa 3°C bis Raumtemperatur instabil.

Patienten mit Niereninsuffizienz können kein EPO bilden und leiden daher an einer Anämie. Versuche, diese EPO-Unterversorgung durch Verabfolgung von EPO zu komplettieren und die Symptome der Anämie zu verringern, sind bereits erfolgreich gewesen. Weitere klinische Anwendungen bestehen in der Verabreichung von hEPO bei iatrogener Anämie nach Chemotherapie oder Strahlentherapie maligner Erkrankungen.

Eine Einzeldosis von EPO beträgt nur wenige Mikrogramme. Aufgrund der kurzen Halbwertszeit nach i.v.- Verabreichung können physiologische Plasma-Spiegel am besten durch subkutane Injektion erreicht werden.

Die bisher vorgeschlagenen oder verfügbaren galenischen Formulierungen von Erythropoietin enthalten Detergens-, und/oder Protein-, Zucker- bzw. Polyalkoholzusätze, die einerseits EPO stabilisieren und andererseits die Adsorption des EPO an die Innenwand des Aufbewahrungsbehälters (d.h. der Ampulle) verhindern sollen (EP-B-0 178 576; EP-A-0 178 665; G. Krystal et al., Blood Bd.67 (1986), 1, 71 - 79). Subkutane oder i.m. Applikation von solchermaßen stabilisiertem EPO führt zu lokalen Entzündungen unter Bildung von Granulomen. Nach bekannten Verfahren hergestelltes hochreines EPO (EP-A-0 236 059; US-A 4,667,159, PCT/US 86/01342 (= WO 86/07594)) verlor innerhalb einer Woche mehr als 25 % seiner Aktivität bei Lagerung bei 24°C. Aus der EP-B-0 178 576 ist ferner eine Lösung von humanem Erythropoietin, das mit ¹⁴C-Formaldehyd reduktiv methyliert wurde, in PBS bekannt; vgl. Experiment 1 und 2.

Der Erfindung liegt die Aufgabe zugrunde, galenische wäßrigen Formulierungen von gereinigtem Erythropoietin bereitzustellen, die frei von den üblichen stabilisierenden Zusätzen sind und trotzdem eine ausreichende Stabilität bei Temperaturen von etwa 3°C bis Raumtemperatur aufweisen, und sich insbesondere zur subkutanen bzw. intramuskulären Applikation eignen.

Überraschenderweise wurde gefunden, daß gereinigtes Erythropoietin (etwa 99%-ig rein), insbesondere gereinigtes natives oder rekombinantes humanes Erythropoietin, in einem physiologisch verträglichen wäßrigen Phosphatpuffer vom pH-Wert 6 bis 8, der ein physiologisch verträgliches Alkalimetallhalogenid, aber sonst keine stabilisierenden Zusätze enthält, in versiegelten Reagenzgläsern oder Glasampullen über lange Zeit bei Temperaturen von 4 bis 8°C stabil ist.

Die Erfindung betrifft somit galenische wäßrige Formulierungen von gereinigtem Erythropoietin in einem physiologisch verträglichen wäßrigen Phosphatpuffer vom pH-Wert 6 bis 8, der ein physiologisch verträgliches Alkalimetallhalogenid, aber sonst keine stabilisierenden Zusätze enthält. Besonders bevorzugt ist ein Puffer aus 50 mM Natriumphosphat, 100 mM NaCl, pH 7,8. Die Konzentration des Erythropoietins in der wäßrigen Pufferlösung beträgt 50 - 1000 »g pro ml Pufferlösung.

Beispiele für physiologisch verträgliche wäßrige Phosphatpuffer sind Natriumphosphatpuffer und Kaliumphosphatpuffer, vorzugsweise Natriumphosphatpuffer. Beispiele für physiologisch verträgliche Alkalimetallhalogenide sind Natriumchlorid und Kaliumchlorid, vorzugsweise Natriumchlorid. Ein bevorzugter Phosphatpuffer ist ein Puffer mit 50 mM Natriumphosphat, 100 mM NaCl, pH 7,8. Dieser Puffer wird abgekürzt auch als PBS bezeichnet. Die wäßrigen Lösungen des Erythropoietins in PBS sind sehr gut zur subkutanen oder intramuskulären Applikation geeignet.

Die Erfindung betrifft zusätzlich die Verwendung der galenischen wäßrigen Formulierungen zur Herstellung von Injektionspräparaten zur subkutanen oder intramuskulären Applikation. Besonders bevorzugt ist die subkutane Verabreichung, weil sie gegenüber den bekannten Formulierungen, die mit Proteinen stabilisiert sind, keine Reizungen und entzündlichen Schmerzen verursacht. Dies haben klinische Versuche ergeben.

Die Beispiele erläutern die Erfindung .

### Beispiel 1

### Reinigung von rh EPO

Die Reinigung von rh EPO erfolgte ausgehend von serumhaltigem oder serumfreiem Medium, das durch rh EPO produzierende animale Zellen konditioniert war, nach dem in der EP-A 0 267 678, Seite 6, Zeile 45, bis Seite 9, Zeile 5, beschriebenen Verfahren. Das Verfahren umfaßt
(1) die Klärung, das Konzentrieren und die Dialyse des Kulturmediums,
(2) die Ionenaustauschchromatographie,
(3) die präparative "reverse phase HPLC" und
(4) die Gelfiltrationschromatographie.

Zur Gelfiltrationschromatographie wurde die Säule mit PBS, d.h. 50 mM Natriumphosphatpuffer, 100 mM NaCl, pH 7,8 äquilibriert. Mit dieser Pufferlösung wurde rh EPO in einem einzigen symmetrischen Peak eluiert (Messung des Eluats bei 280 nm). Das erhaltene rh EPO war mindestens 99%-ig rein gemäß SDS-PAGE.
(5) Verdünnung

Die vereinigten rh EPO-Fraktionen des Gelfiltrationsschritts (4) hatten einen Gehalt von 0,1 - 0,8 mg EPO pro ml und wurden mit dem Natriumphosphat-Natriumchloridpuffer (PBS) pH 7,8 in Einzeldosen zu 100 »g/ml abgefüllt.

### Beispiel 2

### Prüfung der Stabilität

Die Stabilität der gemäß Beispiel 1 erhaltenen PBS-Lösung von rh EPO wurde nach Lagerung der Einzeldosen in sterilen Glasröhrchen im Vergleich mit verschiedenen Stabilisatoren geprüft. Die Aktivität des rh EPO wurde durch ³T-Einbau in mit Phenylhydrazin behandelten Milzzellen der Maus in üblicher Weise gemessen. Die nachfolgend in der Tabelle dargestellten Daten sind bezogen auf die Ausgangsaktivität des eingesetzten rh EPO = 100 %.
(a) Lagerung bei 4 - 8°C

| Stabilisator | Menge an Stabilisator (Gew./Gew. relativ zu EPO) bezogen auf 100 »g EPO/ml PBS | % Aktivität nach 12 Monaten |
|---|---|---|
| PBS allein | - | 78,5 |
| PBS + Sorbit | 5000 | 51 |
| PBS + Glycerin | 6150 | 0 |
| PBS + Haemaccel^{R} | 100 | 68 |

(b) Lagerung bei 37°C (beschleunigter Test)

| Stabilisator | Menge an Stabilisator | % Aktivität nach Monaten | | | |
|---|---|---|---|---|---|
| | | 1 | 2 | 3 | 6 |
| PBS allein | - | 86,3 | 39 | 11,2 | 9,9 |
| PBS + Sorbit | 5000 | 33,5 | 10,7 | 5,1 | 0 |
| PBS + Haemaccel^{R} | 100 | 42 | 14,8 | 6,0 | 5,1 |

Anm.: Haemaccel^{R} ist eine abgebaute Gelatine
Aus den Tabellen ist die überraschend bessere Stabilisierung von rh EPO in PBS (pH 7,8) ohne weiteren Zusatz eines Stabilisators ersichtlich.

## Patentansprüche (Patentansprüche für folgende(n) Vertragsstaat(en): AT, BE, CH, DE, DK, FR, GB, IT, LI, LU, NL, SE)

1. Galenische wäßrige Formulierungen von gereinigtem Erythropoietin in einer physiologisch verträglichen wäßrigen Pufferlösung vom pH-Wert 6 bis 8, die ein physiologisch verträgliches Alkalimetallphosphat und Alkalimetallhalogenid, aber sonst keine stabilisierenden Zusätze enthält.

2. Formulierung nach Anspruch 1, dadurch gekennzeichnet, daß die wäßrige Pufferlösung aus 50 mM Natriumphosphat, 100 mM NaCl, pH 7,8, besteht.

3. Formulierung nach Anspruch 1 oder 2, dadurch gekennzeichnet, daß das Erythropoietin humanes natives oder rekombinantes Erythropoietin ist.

4. Formulierung nach einem der Ansprüche 1 bis 3, dadurch gekennzeichnet, daß die Konzentration des Erythropoietins 50 - 1000 »g pro ml Pufferlösung beträgt.

5. Verwendung einer wäßrigen Formulierung von gereinigtem Erythropoietin gemäß einem der Ansprüche 1 bis 4 zur Herstellung von Injektionspräparaten.

6. Verfahren zur Herstellung einer stabilen galenischen Formulierung von Erythropoietin nach Anspruch 1, dadurch gekennzeichnet, daß Erythropoietin einer Reinheit von ≧ 99 % in einer physiologisch verträglichen wäßrigen Pufferlösung vom pH-wert 6 bis 8 gelöst wird, wobei besagte Pufferlösung ein physiologisch verträgliches Alkalimetallphosphat und Alkalimetallhalogenid, aber sonst keine stabilisierenden Zusätze enthält.

## Patentansprüche (Patentansprüche für folgende(n) Vertragsstaat(en): ES, GR)

1. Verfahren zur Herstellung einer stabilen galenischen Formulierung von Erythropoietin (EPO), dadurch gekennzeichnet, daß EPO einer Reinheit von ≧ 99 % in einer physiologisch verträglichen wäßrigen Pufferlösung vom pH - Wert 6 bis 8 gelöst wird, wobei besagte Pufferlösung ein physiologisch verträgliches Alkalimetallphosphat und Alkalimetallhalogenid, aber sonst keine stabilisierenden Zusätze enthält.

2. Verfahren nach Anspruch 1, dadurch gekennzeichnet, daß die wäßrige Pufferlösung aus 50 mM Natriumphosphat, 100 mM NaCl, pH 7.8 besteht.

3. Verfahren nach Anspruch 1 oder Anspruch 2, dadurch gekennzeichnet, daß das EPO humanes natives oder rekombinantes EPO ist.

4. Verfahren nach einem der Ansprüche 1 bis 3, dadurch gekennzeichnet, daß die Konzentration des EPO 50 - 1000 »g pro ml Pufferlösung beträgt.

5. Verfahren nach einem der Ansprüche 1 bis 4, dadurch gekennzeichnet, daß die galenische Formulierung zur Injektion vorbereitet ist.

## Claims (Claims for the following Contracting State(s): AT, BE, CH, DE, DK, FR, GB, IT, LI, LU, NL, SE)

1. An aqueous pharmaceutical formulation of purified erythropoietin in a physiologically tolerated aqueous buffer solution of pH 6 to 8 which contains a physiologically tolerated alkali metal phosphate and alkali metal halide, but otherwise no stabilizing additives.

2. A formulation as claimed in claim 1, wherein the aqueous buffer solution is composed of 50 mM sodium phosphate, 100 mM NaCl, pH 7.8.

3. A formulation as claimed in claim 1 or 2, wherein the erythropoietin is human native or recombinant erythropoietin.

4. A formulation as claimed in any of claims 1 to 3, wherein the concentration of erythropoietin is 50 - 1000 »g per ml of buffer solution.

5. The use of an aqueous formulation of purified erythropoietin as claimed in any of claims 1 to 4 for preparing injection products.

6. A process for preparing a stable pharmaceutical formulation of erythropoietin as claimed in claim 1, which comprises dissolving erythropoietin with a purity of ≧ 99% in a physiologically tolerated aqueous buffer solution of pH 6 to 8, said buffer solution containing a physiologically tolerated alkali metal phosphate and alkali metal halide, but otherwise no stabilizing additives.

## Claims (Claims for the following Contracting State(s): ES, GR)

1. A process for preparing a stable pharmaceutical formulation of erythropoietin (EPO), which comprises dissolving EPO with a purity of ≧ 99% in a physiologically tolerated aqueous buffer solution of pH 6 to 8, said buffer solution containing a physiologically tolerated alkali metal phosphate and alkali metal halide, but otherwise no stabilizing additives.

2. The process as claimed in claim 1, wherein the aqueous buffer solution is composed of 50 mM sodium phosphate, 100 mM NaCl, pH 7.8.

3. The process as claimed in claim 1 or claim 2, wherein the EPO is human native or recombinant EPO.

4. The process as claimed in any of claims 1 to 3, wherein the concentration of EPO is 50 - 1000 »g per ml of buffer solution.

5. The process as claimed in any of claims 1 to 4, wherein the pharmaceutical formulation is prepared for injection.

## Revendications (Revendications pour l'(les) Etat(s) contractant(s) suivant(s): AT, BE, CH, DE, DK, FR, GB, IT, LI, LU, NL, SE)

1. Formes galéniques aqueuses d'érythropoïétine purifiée, dans une solution aqueuse tampon physiologiquement acceptable à pH 6-8, qui contient un phosphate de métal alcalin physiologiquement acceptable et un halogénure de métal alcalin, mais par ailleurs ne contient aucun additif stabilisant.

2. Forme galénique selon la revendication 1, caractérisée en ce que la solution aqueuse tampon est constituée de 50 mM de phosphate de sodium, 100 mM de NaCl, pH 7,8.

3. Forme galénique selon la revendication 1 ou 2, caractérisée en ce que l'érythropoïétine est une érythropoïétine humaine native ou recombinante.

4. Forme galénique selon l'une des revendications 1 à 3, caractérisée en ce que la concentration de l'érythropoïétine va de 50 à 1 000 »g par ml de solution tampon.

5. Utilisation d'une forme galénique aqueuse d'érythropoïétine purifiée selon l'une des revendications 1 à 4, pour la préparation de compositions injectables.

6. Procédé pour la préparation d'une forme galénique stable d'érythropoïétine selon la revendication 1, caractérisé en ce que l'érythropoïétine, à un degré de pureté ≧99 %, est dissoute dans une solution aqueuse tampon physiologiquement acceptable ayant un pH de 6 à 8, ladite solution tampon contenant un phosphate de métal alcalin physiologiquement acceptable et un halogénure de métal alcalin, mais par ailleurs ne contenant aucun additif stabilisant.

## Revendications (Revendications pour l'(les) Etat(s) contractant(s) suivant(s): ES, GR)

1. Procédé pour la préparation d'une forme galénique stable d'érythropoïétine (EPO), caractérisé en ce que l'EPO, à un degré de pureté ≧99 %, est dissoute dans une solution aqueuse tampon physiologiquement acceptable ayant un pH de 6 à 8, ladite solution tampon contenant un phosphate de métal alcalin physiologiquement acceptable et un halogénure de métal alcalin, mais par ailleurs ne contenant aucun additif stabilisant.

2. Procédé selon la revendication 1, caractérisé en ce que la solution aqueuse tampon est constituée de 50 mM de phosphate de sodium, 100 mM de NaCl, pH 7,8.

3. Procédé selon la revendication 1 ou 2, caractérisé en ce que l'EPO est de l'EPO humaine native ou recombinante.

4. Procédé selon l'une des revendications 1 à 3, caractérisé en ce que la concentration de l'EPO va de 50 à 1 000 »g par ml de solution tampon.

5. Procédé selon l'une des revendications 1 à 4, caractérisé en ce que la forme galénique est préparée pour injection.
